# EUROPEAN PATENT APPLICATION

(11) **EP 4 357 777 A2**
(43) Date of publication of application: **24.04.2024**
(21) Application number: 24156162.0
(22) Date of filing: 05.10.2015
(51) Int. Cl.: G01N 33/483

(54) **METHOD FOR PREDICTING CANCER PROGRESSION BY NANOMECHANICAL PROFILING**

(30) Priority: 03.10.2014 EP 14187685
(62) Divisional of application: 15771989.9
(71) Applicant: Universität Basel, 4001 Basel (CH)
(72) Inventor: LOPARIC, Marko, 4056 Basel (CH); PLODINEC, Marija, 4056 Basel (CH); OBERMANN, Ellen, 4053 Basel (CH); RAEZ, Christian, 4310 Rheinfelden (CH)
(74) Representative: Schulz Junghans Patentanwälte PartGmbB

(57) **Abstract**

The invention relates to a method for staging metastatic potential of a primary tumour sample or related lymph node by nanomechanical measurement and/or for determining the reoccurrence or incidence potential.

## Description

### Background

Breast cancer is the most frequent occurring malignancy and the second most frequent cause of cancer death in women in developed countries. Yet, while primary tumours are rarely fatal, metastases are responsible for the majority of cancer-related deaths. There are some parameters which serve as prognostic markers for the development of metastases, but despite considerable efforts, it is still not possible to predict accurately an individual's risk. Therefore, adjuvant therapy is frequently administered to patients who might have been cured by surgery and anti-hormonal treatment alone. Current classification of breast cancer - including prognostic and predictive markers - is still mainly based on clinical and histopathological criteria, i.e. patient age, tumour size, lymph node involvement, histological type of the tumour, expression of oestrogen- and progesteronreceptors, HER2/neu, and Ki-67 and tumour grade.

Yet risk stratification based on only clinical pathological parameters may be misleading. Especially in early, HER2/neu-negative breast cancer (i.e. stages I, IIA, IIB, and IIIA), these clinicopathological factors are not sufficient for clinical decision making particularly regarding adjuvant chemotherapy since substantial over- or undertreatment may occur. Since 2007, international guidelines have recommended to add further tests to the established risk assessment.

The main goal of the research on breast cancer is therefore the development of prognostic markers which are assessed by quality assured certified tests, can be routinely used, and whose costs are acceptable. These markers should help to optimize cancer diagnosis, orientate therapy choice, and support patient follow-up.

However, all currently available tests suffer from certain drawbacks. In general, they are only validated for well-defined subgroups of patients, for example, in node-negative patients with moderately differentiated tumours. In addition, the tests are mostly performed in central institutions, which require time for delivery of the tumour samples to the central laboratory. In addition, there is no local (quality) control over the processing of the sample. In addition to genetic and microenvironmental factors, recent data show that physical interactions of cancer cells with their environment are critical parameter in the metastatic process. Nevertheless, efforts to understand cancer biomechanics had been largely polarized between tissue-level and single-cell experimentation. As a result, findings have been disputed due to: 1) a lack of the natural tissue context, and 2) insufficient measurements/analysis to account for intratumoural heterogeneities. This highlights that investigating entire tissue segments with sub-cellular resolution provides a more comprehensive understanding of mechanical changes associated with carcinogenesis. This motivated us to develop an atomic force microscope (AFM)-based diagnostic apparatus known as ARTIDIS^{®} (*"Automated and Reliable Tissue Diagnostics"*; US 8,756,711 B2, US 2014338073 A1, WO 2014090971 A1, WO 2015001119 A1, WO 2015018865 A1 incorporated herein by reference) to measure the stiffness profiles of unadulterated tissue biopsies from human patients in close to native physiological conditions with an unprecedented stiffness sensitivity resolved at nanometre-scale spatial resolution. Lasting ~2 hours, an ARTIDIS assay uses a ~10 nm-sharp stylus or tip that makes -10'000 miniscule indentations across a biopsy surface.

Based on this background, it is therefore the objective of the present invention to provide a reliable and simple method for cancer staging and/or providing prognostic and/or predictive information about cancer.

The objective is attained by the subject matter of the independent claims.

### Definitions

The terms *stiffness* or *elasticity* in the context of the present specification refers to the resistance of a tissue sample or tissue to deformation by an applied force. The stiffness or elasticity is measured as the elastic modulus of the tissue sample in Pascal (Pa). A soft tissue sample is characterized by a low stiffness value and a rigid tissue is characterized by an elevated stiffness value.

Such deformation force may be applied to the tissue sample or tissue by a stylus (as part, for example, of an atomic force microscope) that impinges the tissue sample or tissue, wherein either the stylus or the tissue sample is moved in a vertical direction relative to each other. To measure a plurality of points on a sample, the stylus or the sample may be additionally moved in a lateral direction, wherein a lateral direction in the sense of the invention means a direction that is orthogonal to the vertical direction.

The stylus may be a cantilever with a sharp tip or an attached colloidal particle that acts as a probe. A cantilever in the sense of the invention means a beam or arm that is anchored at only one end. Deflections of the cantilever caused by repulsive or attractive forces between the sample surface and the tip may be optically detected, for example by an interferometer or by a laser focused on the cantilever's back, and reflected onto a split photodiode, wherein the photodiode registers the deflection of the cantilever as a voltage difference, which can be converted into nanometres. Alternatively, the deflection of the cantilever may be detected by a piezoelectric sensor, wherein the strain of the cantilever is converted into an electrical charge. Also alternatively, a self-sensing cantilever may be used such as Piezo-Resistive Sensing Active (PRSA) probes, which are for example, silicon cantilevers with an integrated piezo-resistor bridge and a thermal heater. Advantageously, with such cantilevers no laser adjustment is necessary.

The term *area* in the context of the present specification refers to an area that is defined by a grid of (measurement) points, wherein each point corresponds to indentation footprint of the stylus as described above and each point is not more than 100 µm, preferably 50 µm, 20 µm, 10 µm or 1 µm away from its next point. By way of non-limiting example, an area has a size of 25 µm², 50 µm², 100 µm², 200 µm², 300 µm², 400 µm², 500 µm², 600 µm², 750 µm², 1000 µm², 5000 µm² or 10.000 µm² and the geometrical centre points of two areas are at least 100 µm, 200 µm, 300 µm, 400 µm, 500 µm or 1 mm apart.

Measured force and indentation depth for any given sample depend on the cantilever spring constant and tip radius.

The term *spatial resolution* in the context of the present specification refers to the minimal distance between two points on a tissue or tissue sample by which the two points can be discriminated regarding their stiffness. A spatial resolution of at least 1mm, preferably 100 µm, 10 µm or 1 µm means that the maximal distance by which two points still can be discriminated is 1mm, preferably 100 µm, 10 µm or 1 µm. A spatial resolution of at least 100 µm, preferably 10 µm or 1 µm also encompasses higher resolutions. A resolution higher than 1 µm means two points having a distance smaller than 1 µm still can be discriminated. Examples of resolutions higher than 100 µm are 10 µm and 1 µm. Examples of resolutions higher than 1 µm are 0.5 µm, 0.1 µm and 10 nm.

The term *tissue sample* in the context of the present specification refers to a tissue sample that comprises contiguous cells and extracellular matrix. Such tissue sample may be obtained by a biopsy or resection.

The term *resection specimen* in the context of the present specification refers to a sample representing at least a part of an organ or the body that have been removed from the organ or body. A resection specimen may also comprise a whole organ or body part.

The term *tissue biopsy sample* in the context of the present specification refers to a tissue sample that is obtained by a biopsy and comprises contiguous cells and extracellular matrix.

The term *biopsy* in the context of the present specification refers to a method for removal of a tissue part or a tissue for examination. Such biopsy may a needle aspiration biopsy, a punch biopsy, a vacuum-assisted core biopsy, a core needle biopsy or a forceps biopsy. The removal may be performed with the help of suitable tools such as a hollow needle, a round sharp knife or a scalpel. A tissue biopsy sample may additionally be obtained by endoscopes or endoscopic methods.

The biopsy procedure may be guided by a suitable method such as ultrasound or CT (X-ray computed tomography), wherein a tumour or a conspicuous lesion can be detected or located.

The term *normal tissue* in the context of the present specification refers to an ensemble of contiguous cells and extracellular matrix with identically physiological function that are characterized by a normal, controlled growth and normal cellular and extracellular function and structure.

The term *tumour* in the context of the present specification refers to a neoplasm or a lesion that is formed by an abnormal growth of neoplastic cells. The tumour can be benign, premalignant or malignant. The classification of a tissue biopsy samples from a human mammary carcinoma is preferred. The term *benign* lesion or tumour in the context of the present specification refers to a tumour that lacks the ability to metastasize.

The term *primary tumour* in the context of the present specification refers to a tumour originating from the same tissue type as surrounding organ or tissue.

The terms *metastasis* or *metastases* in the context of the present specification refers to tumours which have spread from the primary tumour to distant sites (e.g. different organ).

The terms *malignancy* or "a *malignant tumour"* in the context of the present specification refers to the ability of a tumour to penetrate the basal membrane, invade neighbouring tissues or spread through the body. A malignant tumour is synonymous with a malignant neoplasm or cancer, in particular with invasive cancer.

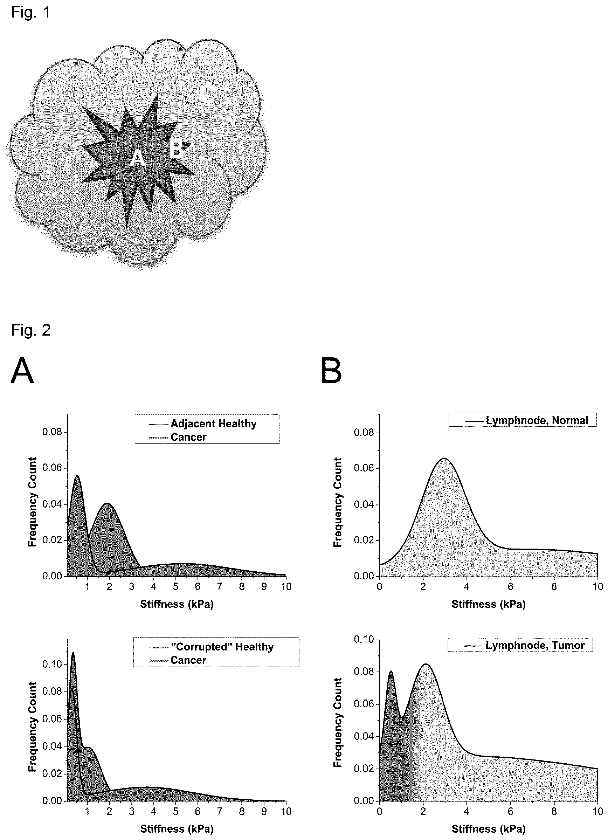

The term *border of the tumour* in the context of the present specification is defined as round, smooth, well-defined (mostly in benign tumour) or irregular, poorly defined (often the case in malignant tumours) border between a tumour and adjacent tissue. Histologically is defined as the outermost part of the tumour where tumour cells can be found (Figure 1B).

The term *adjacent tissue* in the context of the present specification is defined as part of the tissue or organ other than tumour. (Figure 1C). Adjacent tissue is typically surrounding the primary tumour but can be also considered as any part of the tissue of organ without tumour presence.

The term *adjacent lymph node* or *lymph node adjacent to a primary tumour* in the context of the present specification particularly refers to lymph node that drains a tumour. Such adjacent lymph nodes are also referred as to *sentinel lymph nodes.*

The term *axillary lymph node* in the context of the present specification refers to a lymph node that drains lymph vessels from the lateral quadrants of the breast, the superficial lymph vessels from the walls of the chest and the abdomen above the level of the navel, and the vessels from the upper limb. Axillary lymph nodes are also referred to as armpit lymph nodes.

The term *stiffness distribution* in the context of the present specification refers to a frequency of different stiffness values determined from an individual tissue biopsy sample. A determined stiffness distribution may additionally be fitted to a Gaussian function. A unimodal stiffness distribution is a distribution of discrete stiffness values having a single maximum, which indicates a sample having a uniform stiffness. A bimodal distribution function has two maxima. Such distribution may be caused by a sample having two differently stiff parts, for example a soft tumour core and a stiff periphery. A trimodal stiffness distribution in the sense of the invention means a distribution characterized by three local maxima. A trimodal distribution may indicate that normal tissue, a border region characterized by hard stroma and a soft tumour core have contributed to the values making up the distribution. A sample at least bimodal stiffness distribution has a bimodal, trimodal or n-modal (with n being an integer >1) distribution function.

The term *heterogeneous stiffness distribution* in the context of the present specification refers to an n-modal distribution function (with n being an integer >1).

A plurality of stiffness values in the context of the present specification refers to at least 100, 200, 300, 400, 500 900, 1000, 1600, 2500, 3600, 4900, 6400, 8100 or 10000 stiffness values.

A *prognostic marker* provides a risk of cancer incidence and/or recurrence or in other words gives an indication of likelihood of disease progression.

A *predictive marker* provides an indication of patient's response to specific treatment.

The term *peak* in the context of the present specification refers to a local maximum in the stiffness value distribution and signifies the stiffness value with the highest frequency within a sample, or within the immediate neighboring values.

In the context of the present specification, the term *frequency maximum* when used with respect to a stiffness value distribution refers to a stiffness value characterized by a local or absolute maximum of the graph plotting the frequency of stiffness values over the stiffness values. Accordingly, a first frequency maximum is the same or substantially the same as a second frequency maximum, if the stiffness value of the first frequency maximum is the same or substantially the same as the stiffness value of the second frequency maximum.

The term *physiological conditions* in the context of the present specification refers to conditions necessary to preserve the structural integrity and mechanical properties of the biopsy tissue sample, maintaining viability of the tissue by any chemicals or physical agents and include in particular that after collection the sample is stored in a physiological buffer such as phosphate buffered saline, Ringer solution, or transplantation buffer such as Custodiol and stiffness determination is performed at 20, 25, 30 or 37°C. The Ringer solution may further be supplemented with glucose and a protease cocktail. Further, stiffness determination of the biopsy tissue sample may be performed within 1 h, 2 h, 6 h, 12 h, 24 h, 48 h or 72 h after collection without changing the mechanical properties of the sample. "Physiological conditions" particularly do not comprise frozen tissue or thawed tissue, or paraffin-embedded samples.

### Description

The present invention is based on the surprising finding that metastatic lesions are characterized by a similar nanomechanical phenotype as the primary tumour, from which the metastatic lesions are originating.

According to a first aspect of the invention, a method for classifying a tissue biopsy sample obtained from a tumour is provided. The method comprises determining the stiffness values for a plurality of points on the sample with a spatial resolution of at least 100 µm (i.e. each of the plurality of points has a distance of 100 µm or less to the neighbouring points), 50 µm, 20 µm, 10 µm or 1 µm, resulting in a stiffness distribution, and assigning the sample to a probability of malignancy.

In certain embodiments, a high probability of being malignant is assigned to a sample showing an at least bimodal stiffness distribution, wherein the at least bimodal stiffness distribution is characterized by a first peak exhibiting an at least two-fold higher stiffness value than a second peak.

In certain embodiments, a sample exhibiting a heterogeneous stiffness distribution having a frequency maximum below 1 kPa, is classified as an invasive cancer specimen or is assigned a high probability of being malignant. In certain embodiments, a sample exhibiting a heterogeneous stiffness distribution having a frequency maximum from 0.3 kPa to 0.8 kPa, is classified as an invasive cancer specimen or is assigned to a high probability of being malignant. In certain embodiments, a sample exhibiting a heterogeneous stiffness distribution having a frequency maximum from 0.4 kPa to 0.8 kPa, is classified as an invasive cancer specimen or is assigned to a high probability of being malignant. In certain embodiments, a sample exhibiting a heterogeneous stiffness distribution having a frequency maximum from 0.4 kPa to 0.7 kPa, is classified as an invasive cancer specimen or is assigned to a high probability of being malignant. In certain embodiments, a sample exhibiting a heterogeneous stiffness distribution having a frequency maximum from 0.3 kPa to 0.6 kPa, is classified as an invasive cancer specimen or is assigned to a high probability of being malignant.

In certain embodiments, a sample exhibiting an exponential decay in the stiffness distribution is classified as tumour tissue. In certain embodiments, a sample exhibiting an exponential decay from 0.4 kPa to 10 kPa in the stiffness distribution is classified as tumour tissue. In certain embodiments, a sample exhibiting an exponential decay from 0.4 kPa to 15 kPa in the stiffness distribution is classified as tumour tissue. In certain embodiments, a sample exhibiting an exponential decay from 0.4 kPa to 20 kPa in the stiffness distribution is classified as tumour tissue.

In certain embodiments, the method of the invention is applied to a first sample and a second sample.

In certain embodiment, the first sample is a primary tumour sample and the second sample is taken from a lymph node adjacent to the sampling site (the primary tumour site), and the second sample is classified as a lymph node metastasis,
- if the first sample and the second sample both show a heterogeneous stiffness distribution having a frequency maximum below 1 kPa, particularly from 0.3 kPa to 0.8 kPa, from 0.4 kPa to 0.8 kPa, from 0.4 kPa to 0.7 kPa, or from 0.3 kPa to 0.6 kPa, and
- the frequency maximum of the second sample is the same as the frequency maximum of the first sample.

In certain embodiments,
- a first frequency (or stiffness) distribution is obtained from a first site of the sample and a second frequency (or stiffness) distribution is obtained from a second site of the sample, and
- the first site corresponds to a part of the tumour histologically classified as tumour tissue, and the second site corresponds to tissue histologically classified as beyond the border of the tumour (adjacent tissue), and
- the first frequency distribution is characterized by a heterogeneous stiffness distribution having a frequency maximum below 1 kPa, particularly from 0.3 kPa to 0.8 kPa, from 0.4 kPa to 0.8 kPa, from 0.4 kPa to 0.7 kPa, or from 0.3 kPa to 0.6 kPa, and
- the tumour sample is classified as having a low probability of having spread to adjacent lymph nodes, if the second frequency distribution is characterized by absence of a stiffness distribution frequency maximum below 1 kPa.

In certain embodiments, the tumour sample is classified as having a high probability of having spread to adjacent lymph nodes if the second frequency distribution maximum is characterized by presence of a stiffness distribution frequency maximum below 1 kPa.

According to an aspect of the invention, a method for classifying a tissue sample obtained from a patient is provided, wherein the tissue sample is suspected to comprise secondary tumour tissue. The method comprises:
- determining a stiffness value for each of a first plurality of points on a primary tumour sample, resulting in a first stiffness distribution,
- determining a stiffness value for each of a second plurality of points on said tissue biopsy sample, resulting in a second stiffness distribution,
   wherein
- the tissue sample is classified as a metastasis if said first stiffness distribution and said second stiffness distribution both show a heterogeneous stiffness distribution having a frequency maximum at substantially the same stiffness value below 1kPa.

In certain embodiments, the tissue sample is a tissue biopsy sample or a resection specimen.

In certain embodiments, both the first and the second pluralities of points are determined with a spatial resolution of at least 100 µm, 50 µm, 20 µm, 10 µm or 1 µm.

In certain embodiments, the tissue sample was taken or obtained from a lymph node, particularly a lymph node adjacent to the sampling site of the tumour sample or an axillary lymph node.

In certain embodiments, a primary tumour sample exhibiting a frequency maximum below 0.5 kPa is classified as metastasized tumour.

According to an aspect of the invention, a method for classifying a tissue sample obtained from a tumour is provided. The method comprises:
- determining a stiffness value for each of a plurality of points on the sample with a spatial resolution of at least 100 µm, 50 µm, 20 µm, 10 µm or 1 µm, resulting in a stiffness distribution,
- assigning a probability of malignancy to the sample,
   wherein
- the method is applied to a first sample and a second sample, the first sample is a primary tumour sample, and the second sample is a sample taken or obtained from a lymph node, particularly a lymph node adjacent to the sampling site of the first sample or an axillary lymph node, and
- the second sample is classified as a lymph node metastasis if the first sample and the second sample both show a heterogeneous stiffness distribution having a frequency maximum below 1kPa, and the frequency maximum of the second sample is the same as the frequency maximum of the first sample.

In certain embodiments, the tissue sample is a tissue biopsy sample or a resection specimen.

In certain embodiments, a primary tumour sample exhibiting a frequency maximum below 0.5 kPa is classified as metastasized tumour or as having a high probability of having spread to the adjacent tissue, particularly to adjacent lymph nodes or axillary lymph nodes.

In certain embodiments, the primary tumour sample, particularly primary tumour biopsy sample, comprises at least a part of the core of the tumour and at least a part of the periphery of the tumour.

In certain embodiments, the primary tumour sample, particularly the primary tumour biopsy sample, represents at least one half of the cross-section of the tumour described above and exhibiting a distinct orientation from core to periphery of the tumour. In certain embodiments, the primary tumour biopsy sample is a cylindrical or prismatic biopsy.

According to an aspect of the invention, a method for classifying a tissue sample obtained from a tumour is provided. The method comprises:
- determining the stiffness values for a plurality of points on the sample with a spatial resolution of at least 100 µm, 50 µm, 20 µm, 10 µm or 1 µm, resulting in a stiffness distribution,
- assigning the sample to a probability of malignancy,
   wherein
- a first frequency or stiffness distribution is obtained from a first site of the sample and a second frequency or stiffness distribution is obtained from a second site of said sample, and
- the first site corresponds to a part of the tumour histologically classified as tumour tissue and the second site corresponds to adjacent tissue, particularly tissue histologically classified as beyond the border of the tumour, and
- the tumour sample is classified as having a low probability of having spread to the adjacent tissue, particularly to adjacent lymph nodes or axillary lymph nodes, if the first frequency or stiffness distribution is characterized by a heterogeneous stiffness distribution having a frequency maximum below 1kPa, and the second frequency or stiffness distribution is characterized by absence of a stiffness distribution frequency maximum below 1kPa, and/or
- the tumour sample is classified as having a high probability of having spread to the adjacent tissue, particularly to adjacent lymph nodes or axillary lymph nodes, if the second frequency or stiffness distribution is characterized by presence of a stiffness distribution frequency maximum below 1kPa.

In certain embodiments, the tissue sample is a tissue biopsy sample or a resection specimen.

In certain embodiments, a tumour sample exhibiting a frequency maximum below 0.5 kPa, particularly in the first frequency or stiffness distribution, is classified as having a high probability of having spread to the adjacent tissue, particularly to adjacent lymph nodes.

In certain embodiments, the tumour sample comprises the core and the periphery or border of a primary tumour and tissue adjacent to the primary tumour, and optionally one or more adjacent lymph nodes or axillary lymph nodes.

In certain embodiments, the plurality of points is arranged as a grid of n₁ by n₂ points, the grid defining an area, wherein n₁ and n₂ are independently from each other integers >1.

In certain embodiments, a grid of 5 by 5 points (resulting in 25 points), 7 by 7 points, 10 by 10 points, 15 by 15 points, 20 by 20 points, 50 by 50 points or 100 by 100 points are measured for one area. In certain embodiments, the area is defined of a grid of 24x24 points with a size of 400 µm².

In certain embodiments, the stiffness values of at least two different areas of the same sample are determined, and the distance between the geometrical centres of the areas is a multiple of the spatial resolution, said multiple being at least 10 times the spatial resolution. In certain embodiments, the multiple is 20, 30 or 50.

In certain embodiments, the areas of the biopsy sample are positioned on the surface of the sample along the sample's longitudinal axis over a distance of 1 mm, 2 mm, 3 mm, 4 mm, 5 mm, 6 mm, 7 mm, 8 mm, 9 mm, 10 mm, 15 mm or 20 mm.

In certain embodiments, the plurality of points comprises 100, 400, 900, 1000, 1600, 2500, 3600, 4900, 6400, 8100, 10000 or 20000 stiffness values.

In certain embodiments, a sample showing a unimodal stiffness distribution is assigned to a high probability of being non-malignant.

In certain embodiments, the tissue biopsy sample is a cylindrical or prismatic biopsy with a diameter of at least 7 µm. In certain embodiments, the biopsy tissue sample is a cylindrical or prismatic biopsy with a diameter of at least 0.5 mm.

In certain embodiments, the tumour is a human mammary carcinoma or a lymph node, lung, bone, liver or, brain metastasis.

In certain embodiments, the stiffness values are determined under physiological conditions.

In certain embodiments, the stiffness of a mammary biopsy sample is determined and
- a sample showing a stiffness distribution characterized by a peak between 1.1 kPa and 1.5 kPa is assigned to a high probability of being a normal mammary tissue,
- a sample showing a stiffness distribution characterized by a peak between 1.9 kPa and 3.7 kPa is assigned to a high probability of being a benign lesion, and
- a sample showing a stiffness distribution characterized by peaks between 0.31 kPa and 0.75 kPa and at a value larger than 1.2 kPa is assigned to a high probability of being a malignant tumour.

In certain embodiments, a sample showing a stiffness distribution characterized by peaks at between 0.31 kPa and 0.75 kPa and 1.2 kPa and 2.0 kPa is assigned to a high probability of being a malignant tumour.

According to another aspect of the invention, a method for staging cancer or prognosing cancer development is provided. The method comprises
- obtaining a first tissue sample from a primary tumour and a second tissue sample, particularly from a tissue adjacent to the primary tumour, particularly within 5 mm to 10 mm of the histological tumour boundary or not more than 5 mm to 10 mm apart from the histological tumour boundary, from an adjacent lymph node or an axillary lymph node,
- determining the stiffness values for a plurality of points on the first biopsy sample and for a plurality of point on the second biopsy sample, with each of the plurality of points being characterized by a spatial resolution of at least 100 µm, 50 µm, 20 µm, 10 µm or 1 µm, resulting in a stiffness distribution for each of the first and second sample,
- assigning to the first sample a probability of malignancy and assigning to the second sample a probability of being invaded by the primary tumour.

In certain embodiments, a first sample obtained from a primary tumour is provided. In certain embodiments, a second sample obtained from a tissue adjacent to the primary tumour, particularly within 5 mm to 10 mm of the histological tumour boundary or not more than 5mm apart from the histological tumour boundary, from an adjacent lymph node or an axillary lymph node is provided.

In certain embodiments, the first sample represents at least one half of the cross-section of the primary tumour described above and exhibiting a distinct orientation from core to periphery of the primary tumour.

In certain embodiments, the first tissue sample and/or the second tissue biopsy sample is a tissue biopsy sample or a resection specimen.

In certain embodiments,
- the first sample is assigned to a high probability of being malignant if the first sample is characterized by an at least bimodal stiffness distribution having a first peak exhibiting an at least two-fold higher stiffness distribution than a second peak, and/or
- the second sample is assigned to a high probability of being invaded by the primary tumour if the second sample is characterized by an at least bimodal stiffness distribution characterized by a first peak exhibiting an at least two-fold higher stiffness value that a second peak (or a heterogeneous stiffness profile).

In certain embodiments, the tissue adjacent to the primary tumour is comprised within a lymph node, particularly an adjacent lymph node (adjacent to the primary tumour) or an axillary lymph node.

In certain embodiments, a first sample exhibiting a second below 0.5 kPa is classified as metastasized tumour.

According to another aspect of the invention, a method for staging cancer and/or determining cancer incidence is provided. The method comprises
- obtaining a tissue sample from a tissue adjacent to a primary tumour,
- determining the stiffness values for a plurality of points on the sample with a spatial resolution of at least 100 µm, 50 µm, 20 µm, 10 µm or 1 µm, resulting in a stiffness distribution,
- assigning the sample to a probability of being invaded by the primary tumour.

In certain embodiments, a tissue sample from a tissue adjacent to a primary tumour is provided.

In certain embodiments, a high probability is assigned to the sample of being invaded by the primary tumour if the stiffness distribution is characterized by a maximum between 0.2 kPa and 1 kPa, particularly between 0.3 kPa and 0.8 kPa.

In certain embodiments, a sample showing an at least bimodal stiffness distribution is assigned to a high probability of being invaded by the primary tumour, wherein the at least bimodal stiffness distribution is characterized by a first peak exhibiting an at least two-fold higher stiffness value than a second peak.

In certain embodiments, the tissue sample is a tissue biopsy sample or a resection specimen.

According to another aspect, a method for classifying a tissue sample obtained from a tumour is provided, the method comprises:
- determining the stiffness values for a plurality of points on said sample with a spatial resolution of at least 100 µm, 50 µm, 20 µm, 10 µm or 1 µm, resulting in a stiffness distribution,
- assigning to said sample to a probability of malignancy,
   wherein
- a sample showing an at least bimodal stiffness distribution with a peak below 0.5 kPa is classified as a metastasized tumour.

In certain embodiments, the tissue sample is obtained from a mammary carcinoma, particularly from a human mammary carcinoma.

In certain embodiments, the tissue sample is a tissue biopsy sample or a resection specimen.

In certain embodiments, the above mentioned tumour or the above mentioned primary tumour is a mammary carcinoma, kidney tumour, prostate tumour, brain tumour, lung tumour, ovarian tumour, pancreas tumour, or stomach tumour, liver tumour, skin tumour or gastric tumour.

According to a further aspect of the invention, a device for tumour sample diagnosis is provided. The device comprises an atomic force microscope and a computer connected thereto, the computer being configured to run a program conducting any one the above mentioned methods of the invention.

In certain embodiments of any of the methods described previously, the spatial resolution is 20 µm, 10 µm, 5 µm or 1 µm.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

Short description of the figures
- Fig. 1: shows A - tumour B - border of the tumour and adjacent tissue C - adjacent tissue.
- Fig. 2: shows the soft nanomechanical profile of the primary tumour and the adjacent tissue reveal a common phenotype detected in the lymph node metastases. (A) Nanomechanical profiles of two cancer patients reveal bimodal distributions with an exponential decay in the case of patient 1 with peak values at 0.39 +/- 0.21 kPa for specific cancer cell population and 3.66 +/- 2.14 kPa decaying up to 10 kPa for the surrounding cellular and stromal components of the tissue. Adjacent tissue with pathohistological characteristics of normal breast tissues exhibits a uniform stiffness peak of 1.9 +/- 0.7 kPa (top). Sharp distribution and overall softer phenotype is measured for patient 2 with peak values for cells at 0.45 +/- 0.18 kPa and 1.05 +/- 0.55 kPa respectively (bottom). Adjacent tissue of this patient shows a bimodal distribution with a very prominent soft peak of 0.4 +/- 0.2 kPa as well a stiffer peak of 1.05 +/- 0.5 kPa. In the pathohistological analysis this tissue is marked as non-malignant. Therefore here we use the term "Corrupted" healthy since this tissue stiffness values and bimodal distribution do not correspond to the values measured for healthy breast samples from control patients. (B) Patient 1 has cancer negative lymph node exhibiting stiffness values of 3.02 +/- 2.10 kPa (top). Lymph node containing breast cancer metastases from patient 2 exhibits bimodal stiffness distribution with peak values for cancerous region of 0.49 +/- 0.23 kPa that corresponds directly to "soft" cancer cell phenotype found in the primary tumour and the adjacent ("Corrupted" Healthy tissue) while the value of 2.11 +/- 0.78 kPa is measured for the rest of the lymph node (bottom).

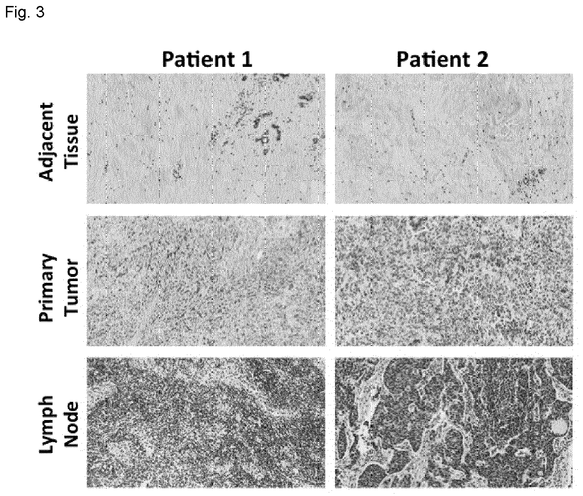

- Fig. 3: shows a post-AFM histological overview of the resection specimens from two breast cancer patients. H&E staining of both samples exhibit similar features; i.e. connective tissue and normal epithelium not significantly different to healthy breast (top). Primary tumours in both cases reveal an invasive breast carcinoma cells with infiltrating nests of cells that have evoked a dense fibrous tissue response (middle). H &E staining of the corresponding lymph node from the patient 2 reveals nests of breast cancer cells similarly to the primary tumour (bottom right), while the lymph node from patient 1 is clear of cancer cells (bottom left).

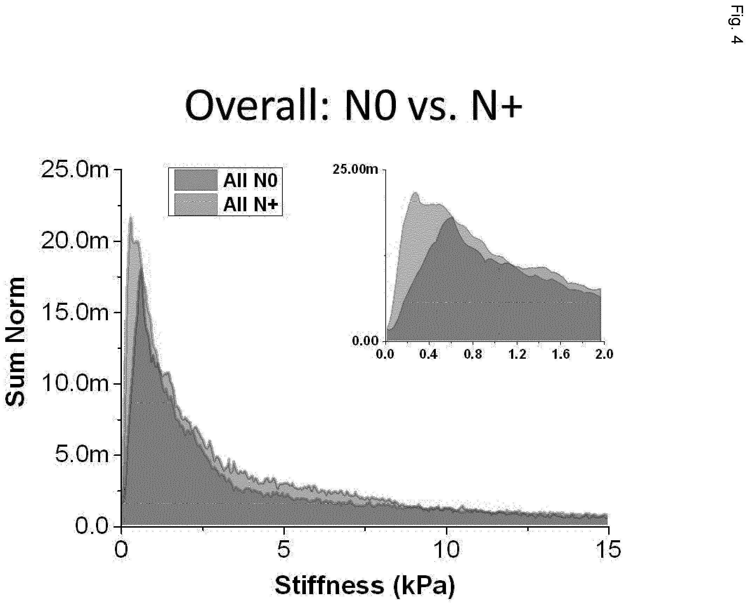

- Fig. 4: shows normalized histograms representing the nanomechanical profile of the primary tumours (biopsy samples and resection specimens) of patient without invaded lymph node N0 (n = 10) and with invaded lymph nodes N+ (n = 11). N+ patients clearly show a shift to softer values in the cancer area (below 1 kPa). They peak at 0.325 kPa, where the N0 patients peak at 0.625 kPa. Both distributions remain quite broad and the part of the histogram above 2 kPa looks very similar in both cases.

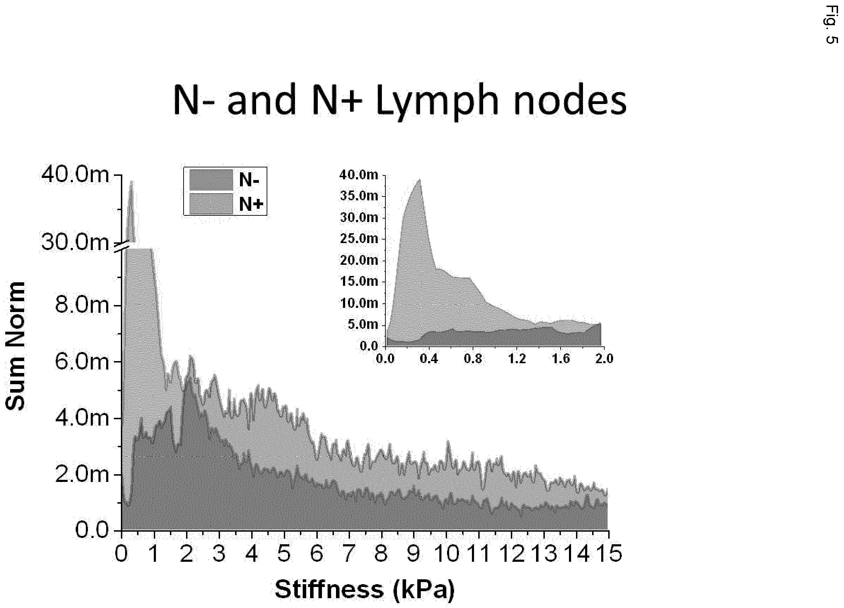

- Fig. 5: shows normalized histograms representing the nanomechnical profile non-invaded and invaded lymph nodes corresponding to the primary tumours illustrated in Fig. 4. Cancer cell positive (N+) lymph nodes have a strong, soft peak around 0.4 kPa, whereas cancer negative (N-) lymph nodes lack a soft peak.

### Examples:

In order to transfer preliminary results into a clinical setting, the ARTIDIS technology was optimized for analysis of unfixed (measured in physiological aqueous environment or frozen tissue) human breast cancer samples obtained by tumour resections. For this purpose, 152 tissue samples, including primary breast cancers of various stage and grade, lymph node metastases, and non-neoplastic human breast tissues were collected from resection specimens of 56 patients undergoing either lumpectomy or mastectomy procedures.

Nanomechanical measurements of the samples were performed as disclosed in US 8,756,711 B2. Briefly, each sample was examined in a systematic manner by homogeneously distributing FV maps over the the whole sample surface to account for possible heterogeneities. A regular distance of approximately 500 µm was kept between the scan using either micrometer screws or automated positioning systems. This resulted in roughly 10 to 15 FV maps per specimen depending on the total biopsy size.

For the analysis of the samples by AFM, biopsies were glued onto a culture dish using 2-component 5-minute fast drying epoxy glue. After a pre-drying step of 2 minutes (to avoid mixing of the epoxy and the specimen buffer), the specimen was laid flat onto the glue in order to optimize the indentation angle and to avoid influence from external components (e.g. the cantilever holder). Pipette tips acting as "ramps" were placed directly under uneven segments of each specimen to maintain height consistency. The use of excessive force (e.g. tearing or stretching) was minimized at all times during specimen handling. All preparative steps were performed in either a sterile buffer environment supplemented with protease inhibitors or transplantation buffer to prevent contamination and to ensuring that the specimen remained in a close-to-in-vivo state. The mounted specimens were kept in ice-cold Ringer's solution or Custodiol until nanomechanical testing, which was performed at room temperature or at 37°C.

For sharp pyramidal tips (205-µm-long silicon nitride cantilevers, nominal cantilever spring constant k = 0.06 N m⁻¹, resonance frequency [air]=18 kHz), the exact spring constant k of the cantilever was determined prior to every experiment with the thermal tune method while the deflection sensitivity was determined in fluid using solid glass substrates as an infinitely stiff reference material.

Contact stiffness (elastic modulus, E) measurements of biopsies were derived as follows; load-displacement curves, also designated as force indentation curves, were recorded at a given site in an oriented manner during both loading and unloading. A regular distance of approximately 500 µm was kept between the scan regions using either micrometer screws or automated positioning systems. An individual set of data consisted of 1,024 load-displacement curves, at an indentation speed of 16 µm/s. This resulted in roughly 15 to 20 force volume maps per sample. When possible, force- volume maps (FV) were made over a 32x32 point grid with a scan size of 20×20µm at a rate of approx. 1 load and unload cycles per second. Each load-displacement curve consisted of at least 512 data points whereas the Z length was set to 5µm to 8 µm depending on the properties of the analyzed region. Each FV map was set to 20 × 20 µm² in order to (i) optimize experimental time as well as (ii) to provide a sufficiently large area incorporating all components within the tissue (e.g., cells and extracellular matrix). The maximum applied loading force was set to 1.8 nN and an indentation depth of approximately 150 to 3000 nm. Additional 72 × 72 FV maps (5184 force-displacement curves per map and a pixel size of 277 nm) were obtained to increase the spatial resolution over key areas of interest.

Force indentation curves were analyzed using a method described previously (Loparic, et al., Biophysical Journal,. 98(11): p. 2731-40, 2010, Plodinec, et al., Journal of Structural Biology,. 174(3): p. 476-484, 2011). Briefly, software was developed in LabVIEW (National Instrument, US) for the automated analysis of the FV data. The contact point was determined. Force curves were obtained transforming from piezo displacement to tip-sample distance, which accounts for the bending of the cantilever and by multiplying cantilever deflection d with the spring constant k to obtain the load F. Unloading force curves were analyzed by performing a linear fit to the upper 50% of the force curve, which defines the stiffness between the maximum load F = 1.8 nN and a load of 0.9 nN. Extraneous effects on the force curve such as adhesion could be avoided by this procedure. The Poisson ratio was set to 0.5. The Young's modulus was determined according to the Oliver and Pharr method (Oliver et al., Journal of Materials Research, 7(6), 1564-1583, 1992). The slope values were spatially plotted, analyzed and displayed in ARTIDIS OFFLINE SOFTWARE.

Post-AFM, tissue samples were fixed and paraffin embedded in an oriented manner. ARTIDIS data have confirmed the initial findings that all carcinoma samples display heterogeneous stiffness phenotypes with a characteristic 2-fold softer phenotype in comparison to the surrounding non-neoplastic and morphologically normal breast tissue. Healthy mammary tissue of patients without breast cancer exhibits on average stiffness values ~1.6 kPa.

Most importantly, the data illustrated in Figure 2 and Figure 3 show:
1) Tumour tissues from breast cancer patients exhibit a heterogeneous distribution from 0.4 kPa and an exponential decay that can range up to 20 kPa. We identified invasive breast cancer specimens by a characteristic soft peak of 0.4 to 0.8 kPa. Stiffness distribution of corresponding lymph node metastases from a same patient was characterized by a heterogeneous stiffness profile with a characteristic soft peak of 0.4 to 0.8 kPa similarly to the primary breast cancer tissue.
2) Adjacent tissue of these patients that was histologically rated as "non-malignant" presented a bimodal distribution with prominent soft stiffness peak ranging from 0.4 to 0.8 kPa. The presence of such tissue that according to nanomechanical analysis is cancerous, but histologically is non-malignant, is an indicator of poor prognosis. In addition stiffness values from 1.2 to 1.9 kPa that correspond to the healthy breast tissue were present as well.
3) In cases where patients had soft stiffness peak detected only in the primary tumour, but no soft peaks in the adjacent tissue, no lymph node metastases were present.
4) In case when fat tissue is measured, specific stiffness peak of 0.2 kPa is present.
5) Because of usual increase of fat component within the breast tissue during aging, very often a fat specific stiffness peak of 0.2kPa is measured.

Additionally, the data illustrate in Fig. 4 and 5 that tumours that have already spread to adjacent lymph nodes show a shift to softer values in the cancer area.

Accordingly, for assessment of cancer aggressiveness and prognosis in breast cancer patients it is important to take into account not just the primary tumour but also the nanomechanical response of the adjacent tissue.

The data presented herein demonstrate applicability of nanomechanical profiling using ARTIDIS in clinics for:
1) Prognosis of cancer incidence, progression and recurrence
2) Prediction of the treatment response
3) Deciding on the appropriate treatment and follow up regimen based on the combined nanomechanical profile of primary tumour and the adjacent tissue
4) Screening of the histologically "non-malignant" tissue (i.e. non-malignant breast tissue with no obvious pathological changes observed with standard screening methods such as ultrasound, mammography or H&E staining after a local biopsy is performed) for the soft stiffness peak with purpose of identifying presence of locally invasive cancer cells and aggressiveness degree of the tumour long before solid tumour growth and presence of symptoms
5) Screening using ARTIDIS nanomechanical profiling is particularly suitable for patients carrying genetic mutations such as BRCA 1 and BRCA2 or any other, who are at high risk for cancer development

The nanomechanical profiling method of the invention is ideally suited for use in daily practice as it allows fast, on-site assessment of specimen and does not suffer from inter-observer variability as for example other markers, such as Ki-67.

The method of the invention is based on the following principles:
1. Tissues (cells and extracellular matrix) undergo mechanical/structural alterations at the nanometer scale before tumour (cancer) starts to develop.
2. Alterations from Point 1 are present across the organ or adjacent tissue (e.g. diffuse/multifocal appearance).
3. The exact location where cancer will first occur depends on the specific local microenvironmental conditions (e.g. alteration and interactions within cells and surrounding extracellular matrix and in-between them).
4. Measurement of nanomechanical profile of the organ/adjacent tissue can detect specific alterations from Point 1.
5. Measurement of nanomechanical profile of the organ/adjacent tissue can distinguish between age related mechanical alteration and pre-tumour, tumour and inflammation related alterations.
6. Measurement of nanomechanical profile of the organ/adjacent tissue can correlate different types and/or degrees of mechanical alterations with tumour ability to progress and/or seed metastases.
7. Results of Point 6 can be used as prognostic and/or predictive marker of tumour development and progression. This can be practically used for better treatment of patients for specific disease.
8. The methodology of the invention is typically applied for detection of breast tumours (e.g. screening) and as prognostic and marker of already existing tumour or tumour associated metastases but is not limited to breast tissue. It can be also used for other organ specific tumours, inflammatory or medically relevant disease like prostate cancer, colorectal cancer, lung cancer, malignant melanoma, liver cancer, dermatitis, gastritis etc.
9. By using the proposed methodology specific conditions: dysplasia, metaplasia, hyperplasia (pre-tumor changes) and age related changes can be specifically detected and distinguished from tumour related changes.

In the following, further aspects of the present invention and embodiments thereof are stated as items. These items may also serve as claims of the application at hand.

Item 1: A method for classifying a tissue sample obtained from a patient, wherein said tissue sample is suspected to comprise secondary tumour tissue, said method comprising
- determining a stiffness value for each of a first plurality of points on a primary tumour sample, resulting in a first stiffness distribution,
- determining a stiffness value for each of a second plurality of points on said tissue sample, resulting in a second stiffness distribution,
   wherein
- said tissue sample is classified as a metastasis if said first stiffness distribution and said second stiffness distribution both show a heterogeneous stiffness distribution having a frequency maximum at substantially the same stiffness value below 1kPa.

Item 2: The method of item 1, wherein both said first and said second pluralities of points are determined with a spatial resolution of at least 100 µm.

Item 3: The method of any one of items 1 or 2, wherein said tissue sample was taken from a lymph node, particularly adjacent to the sampling site of said tumour biopsy sample or an axillary lymph node.

Item 4: A method for classifying a tissue sample obtained from a tumour, comprising
- determining a stiffness value for each of a plurality of points on said sample with a spatial resolution of at least 100 µm, resulting in a stiffness distribution,
- assigning a probability of malignancy to said sample,
   wherein
- said method is applied to a first sample and a second sample, said first sample is a primary tumour sample, and said second sample is a sample taken from a lymph node, particularly a lymph node adjacent to said sampling site of said first sample or an axillary lymph node, and
- said second sample is classified as a lymph node metastasis if said first sample and said second sample both show a heterogeneous stiffness distribution having a frequency maximum below 1kPa, and the frequency maximum of the second sample is the same as the frequency maximum of the first sample.

Item 5: A method for classifying a tissue sample obtained from a tumour, comprising
- determining the stiffness values for a plurality of points on said sample with a spatial resolution of at least 100 µm, resulting in a stiffness distribution,
- assigning to said sample to a probability of malignancy,
   wherein
- a first stiffness distribution is obtained from a first site of said sample and a second stiffness distribution is obtained from a second site of said sample, and
- said first site corresponds to a part of said tumour histologically classified as tumour tissue and said second site corresponds to adjacent tissue, particularly tissue histologically classified as beyond the border of the tumour, and
- said tumour sample is classified as having a low probability of having spread to said adjacent tissue, particularly to adjacent lymph nodes or an axillary lymph node, if said first stiffness distribution is a heterogeneous stiffness distribution having a frequency maximum below 1kPa, and said second frequency distribution is characterized by absence of a stiffness distribution frequency maximum below 1 kPa, and/or
- said tumour sample is classified as having a high probability of having spread to said adjacent tissue, particularly to adjacent lymph nodes or an axillary lymph node, if said second frequency distribution is characterized by presence of a stiffness distribution frequency maximum below 1 kPa.

Item 6: The method according to any one of the preceding items, wherein said tissue sample is a tissue biopsy sample or a resection specimen.

Item 7: The method according to any one of the previous items, wherein said plurality of points is arranged as a grid of n₁ by n₂ points, said grid defining an area.

Item 8: The method according to any one of the previous items, whereby said stiffness values of at least two different areas of said same sample are determined, and the distance between the geometrical centres of said areas is a multiple of said spatial resolution of at least 10.

Item 9: The method according to any one of the preceding items, wherein said plurality of points comprises 100, 400, 900, 1000, 1600, 2500, 3600, 4900, 6400, 8100 or 10000 stiffness values.

Item 10: The method according to any one of the previous items, characterized by that said tissue sample is a cylindrical or prismatic biopsy with a diameter of at least 7 µm.

Item 11: The method according to any one of the previous items, wherein said tumour is a human mammary carcinoma or a lymph node, lung, bone, liver or brain metastasis.

Item 12: The method according to any one of the previous items, characterized by that said stiffness values are determined under physiological conditions.

Iten 13: The method according to any one of the preceding items, wherein a primary tumour sample exhibiting a frequency maximum below 0.5 kPa is classified as metastasized tumour or as having a high probability of having spread to adjacent tissue, particularly to adjacent lymph nodes or to axillary lymph nodes.

Item 14: A method for staging cancer, comprising
- obtaining a first tissue sample from a primary tumour and a second tissue sample,
- determining the stiffness values for a plurality of points on said first tissue sample and for a plurality of points on said second biopsy sample, with each of said plurality of points being characterized by a spatial resolution of at least 100 µm, resulting in a stiffness distribution for each of said first tissue sample and said second tissue sample,
- assigning to said first sample a probability of malignancy, and assigning to said second sample a probability of being invaded by said primary tumour.

Item 15: The method according to item 14, wherein said second tissue sample is obtained from a tissue adjacent to said primary tumour or a lymph node, particularly an adjacent lymph node or an axillary lymph node.

Item 16: The method according to item 14 or 15, wherein said first tissue sample and/or said second tissue sample is a tissue biopsy sample or a resection specimen.

Item 17: The method according to any one of items 14 to 16, wherein
- said first sample is assigned a high probability of being malignant if said first sample is characterized by an at least bimodal stiffness distribution having a first peak exhibiting an at least two-fold higher stiffness value than a second peak, and/or
- said second sample is assigned a high probability of being invaded by said primary tumour if said second sample is characterized by an at least bimodal stiffness distribution characterized by a first peak exhibiting an at least two-fold higher stiffness value than a second peak.

Item 18: The method according to any one of items 14 to 17, wherein said tissue adjacent to said primary tumour is comprised within a lymph node.

Item 19: The method according to any one of items 14 to 18, wherein a first sample exhibiting a second peak below 0.5 kPa is classified as metastasized tumour.

Item 20: A method for staging cancer, comprising
- obtaining a tissue sample form a tissue adjacent to a primary tumour,
- determining the stiffness values for a plurality of points on said sample with a spatial resolution of at least 100 µm, resulting in a stiffness distribution,
- assigning to said sample a probability of being invaded by said primary tumour.

Item 21: The method according to item 20, wherein said tissue sample is a tissue biopsy sample or a resection specimen.

Item 22: The method according to item 20 or 21, wherein said sample is assigned to a high probability of being invaded by said primary tumour if said stiffness distribution is characterized by a maximum between 0,2 kPa and 1 kPa, particularly between 0,3 kPa and 0,8 kPa.

Item 23: The method according to any one of items 20 to 22, wherein a sample showing an at least bimodal stiffness distribution is assigned a high probability of being invaded by said primary tumour, wherein said at least bimodal stiffness distribution is characterized by a first peak exhibiting an at least two-fold higher stiffness value than a second peak.

Item 24: A method for classifying a tissue sample obtained from a tumour comprising
- determining the stiffness values for a plurality of points on said sample with a spatial resolution of at least 100 µm, resulting in a stiffness distribution,
- assigning to said sample to a probability of malignancy,
   wherein
- a sample exhibiting a peak in said stiffness distribution below 0.5 kPa is classified as metastasized tumour.

Item 25: The method according to item 24, wherein said tissue sample is obtained from a mammary carcinoma.

Item 26: The method according to item 24 or 25, wherein said tissue sample is a tissue biopsy sample or a resection specimen.

Item 27: The method according to any one of the preceding items, wherein said primary tumour or said tumour is a mammary carcinoma, kidney tumour, prostate tumour, brain tumour, lung tumour, ovarian tumour, pancreas tumour, or stomach tumour, liver tumour, skin tumour or gastric tumour.

Item 28: A device for tumour sample diagnosis, comprising an atomic force microscope and a computer connected thereto, the computer being configured to run a programme conducting the method of any of the previous items.

Item 29: The method according to any one of the preceding items, wherein said spatial resolution is 20 µm, 10 µm, 5 µm or 1 µm.

## Claims

1. A method for classifying a tissue sample obtained from a patient, wherein said tissue sample is suspected to comprise secondary tumour tissue, said method comprising
- determining a stiffness value for each of a first plurality of points on a primary tumour sample, resulting in a first stiffness distribution,
- determining a stiffness value for each of a second plurality of points on said tissue sample, resulting in a second stiffness distribution,
wherein
- said tissue sample is classified as a metastasis if said first stiffness distribution and said second stiffness distribution both show a heterogeneous stiffness distribution having a frequency maximum at substantially the same stiffness value below 1kPa.

2. The method of claim 1, wherein both said first and said second pluralities of points are determined with a spatial resolution of at least 100 µm.

3. The method of any one of claims 1 or 2, wherein said tissue sample was taken from a lymph node, particularly adjacent to the sampling site of said tumour biopsy sample or an axillary lymph node.

4. A method for classifying a tissue sample obtained from a tumour, comprising
- determining a stiffness value for each of a plurality of points on said sample with a spatial resolution of at least 100 µm, resulting in a stiffness distribution,
- assigning a probability of malignancy to said sample,
wherein
- said method is applied to a first sample and a second sample, said first sample is a primary tumour sample, and said second sample is a sample taken from a lymph node, particularly a lymph node adjacent to said sampling site of said first sample or an axillary lymph node, and
- said second sample is classified as a lymph node metastasis if said first sample and said second sample both show a heterogeneous stiffness distribution having a frequency maximum below 1kPa, and the frequency maximum of the second sample is the same as the frequency maximum of the first sample.

5. A method for classifying a tissue sample obtained from a tumour, comprising
- determining the stiffness values for a plurality of points on said sample with a spatial resolution of at least 100 µm, resulting in a stiffness distribution,
- assigning to said sample to a probability of malignancy,
wherein
- a first stiffness distribution is obtained from a first site of said sample and a second stiffness distribution is obtained from a second site of said sample, and
- said first site corresponds to a part of said tumour histologically classified as tumour tissue and said second site corresponds to adjacent tissue, particularly tissue histologically classified as beyond the border of the tumour, and
- said tumour sample is classified as having a low probability of having spread to said adjacent tissue, particularly to adjacent lymph nodes or an axillary lymph node, if said first stiffness distribution is a heterogeneous stiffness distribution having a frequency maximum below 1kPa, and said second frequency distribution is **characterized by** absence of a stiffness distribution frequency maximum below 1 kPa, and/or
- said tumour sample is classified as having a high probability of having spread to said adjacent tissue, particularly to adjacent lymph nodes or an axillary lymph node, if said second frequency distribution is **characterized by** presence of a stiffness distribution frequency maximum below 1 kPa.

6. The method according to any one of the preceding claims, wherein said tissue sample is a tissue biopsy sample or a resection specimen.

7. The method according to any one of the previous claims, wherein said plurality of points is arranged as a grid of n₁ by n₂ points, said grid defining an area.

8. The method according to any one of the previous claims, whereby said stiffness values of at least two different areas of said same sample are determined, and the distance between the geometrical centres of said areas is a multiple of said spatial resolution of at least 10.

9. The method according to any one of the preceding claims, wherein said plurality of points comprises 100, 400, 900, 1000, 1600, 2500, 3600, 4900, 6400, 8100 or 10000 stiffness values.

10. The method according to any one of the previous claims, **characterized by** that said tissue sample is a cylindrical or prismatic biopsy with a diameter of at least 7 µm.

11. The method according to any one of the previous claims, wherein said tumour is a human mammary carcinoma or a lymph node, lung, bone, liver or brain metastasis.

12. The method according to any one of the previous claims, **characterized by** that said stiffness values are determined under physiological conditions.

13. The method according to any one of the preceding claims, wherein a primary tumour sample exhibiting a frequency maximum below 0.5 kPa is classified as metastasized tumour or as having a high probability of having spread to adjacent tissue, particularly to adjacent lymph nodes or to axillary lymph nodes.

14. A method for staging cancer, comprising
- obtaining a first tissue sample from a primary tumour and a second tissue sample,
- determining the stiffness values for a plurality of points on said first tissue sample and for a plurality of points on said second biopsy sample, with each of said plurality of points being **characterized by** a spatial resolution of at least 100 µm, resulting in a stiffness distribution for each of said first tissue sample and said second tissue sample,
- assigning to said first sample a probability of malignancy, and assigning to said second sample a probability of being invaded by said primary tumour.

15. The method according to claim 14, wherein said second tissue sample is obtained from a tissue adjacent to said primary tumour or a lymph node, particularly an adjacent lymph node or an axillary lymph node.
